# EUROPÄISCHE PATENTSCHRIFT

(11) **EP 3 846 969 B1**
(45) Veröffentlichungstag und Bekanntmachung des Hinweises auf die Patenterteilung: **02.11.2022**
(21) Anmeldenummer: 18769583.8
(22) Anmeldetag: 06.09.2018
(51) Int. Cl.: A61B 90/98, A61B 90/00, B25B 23/14, B25B 15/00, A61B 17/88

(54) **VORRICHTUNG ZUR ÜBERTRAGUNG EINES VORGEGEBENEN DREHMOMENTS AUF EIN CHIRURGISCHES WERKZEUG**
DEVICE FOR TRANSMITTING A SPECIFIED TORQUE TO A SURGICAL TOOL
DISPOSITIF DE TRANSMISSION D'UN COUPLE PRÉDÉTERMINÉ À UN OUTIL CHIRURGICAL

(43) Veröffentlichungstag der Anmeldung: 14.07.2021
(73) Patentinhaber: SMC Innovation GmbH, 6330 Cham (CH)
(72) Erfinder: JANSEN-TROY, Arne, 78333 Stockach (DE); STUDER, Armin, 6330 Cham (CH)
(74) Vertreter: Daub, Thomas
(86) Internationale Anmeldenummer: PCT/CH2018/000036
(87) Internationale Veröffentlichungsnummer: WO 2020/047680

(56) Entgegenhaltungen:
- DE-A1-102014 105 078
- DE-A1-102015 122 568

## Beschreibung

Die Erfindung bezieht sich auf eine Vorrichtung zur Übertragung eines vorgegebenen Drehmoments auf ein chirurgisches Werkzeug gemäss dem Oberbegriff des Patentanspruchs 1, auf eine Kombination gemäss dem Oberbegriff des Patentanspruchs 13, auf ein Kit gemäss dem Oberbegriff des Patentanspruchs 14 und auf ein Verfahren zur Montage der Vorrichtung gemäss dem Oberbegriff des Patentanspruchs 15.

Eine Vorrichtung zur Übertragung eines vorgegebenen Drehmoments auf ein chirurgisches Werkzeug nach dem Oberbegriff von Anspruch 1 ist aus dem Dokument WO 2015/155097 bekannt. Diese bekannte Vorrichtung umfasst eine Drehmomentübertragungseinheit mit mehreren Drehmomentbegrenzungselementen, welche auf einer Längsachse hintereinander angeordnet und jeweils über eine Sollbruchstelle miteinander verbunden sind. Die Drehmomentübertragungseinheit ist drehfest aber axial verschiebbar in einem Schaft angeordnet, wobei ein hinterstes Drehmomentbegrenzungselement über das hintere Ende des Schaftes hinausragt und drehfest mit einem Handgriff verbunden ist. Beim Überschreiten des vorgegebenen Drehmoments wird das hinterste drehfest im Handgriff gelagerte Drehmomentbegrenzungselement an der Sollbruchstelle durch Torsion abgetrennt und in einem Hohlraum im Handgriff aufgefangen. Zugleich wird durch eine am Schaft angeordnete Ladeeinrichtung die Drehmomentübertragungseinheit gegen das hintere Ende des Schaftes verschoben, so dass das nächste Drehmomentbegrenzungselement über das hintere Ende des Schaftes hinausragt und drehfest mit dem Handgriff verbunden ist.

Nachteilig an dieser bekannten Vorrichtung ist, dass die Vorrichtung komplett geliefert wird und nicht demontierbar ist, so dass nach einer begrenzten Anzahl Drehmomentüberschreitungen (maximal 10), d.h. nach einer mechanischen Zerstörung der Drehmomentübertragungseinrichtung die gesamte Vorrichtung nicht mehr weiterverwendet werden kann und entsorgt werden muss.

Hier will die Erfindung Abhilfe schaffen. Der Erfindung liegt die Aufgabe zugrunde, eine Vorrichtung zur Übertragung eines vorgegebenen Drehmoments auf ein chirurgisches Werkzeug zu schaffen, welche so konfiguriert ist, dass ein Auswechseln oder Austauschen von Drehmomentübertragungseinheiten auf einfache Weise ermöglicht wird.

Die Erfindung löst die gestellte Aufgabe mit einer Vorrichtung zur Übertragung eines vorgegebenen Drehmoments auf ein chirurgisches Werkzeug, welche die Merkmale des Anspruch 1 aufweist, mit einer Kombination, welche die Merkmale des Anspruchs 13 aufweist, mit einem Kit, welcher die Merkmale des Anspruchs 14 aufweist, sowie mit einem Verfahren zur Montage der Vorrichtung, welches die Merkmale des Anspruchs 15 aufweist.

Die durch die Erfindung erreichten Vorteile sind im wesentlichen darin zu sehen, dass dank der erfindungsgemässen Vorrichtung:
- eine verbrauchte, d.h. zerstörte Drehmomentübertragungseinheit mittels Entfernen und Wiedereinsetzen des Einsatzes durch wenige Handgriffe auf einfache Weise durch eine neue ungebrauchte Drehmomentübertragungseinheit ersetzt werden kann;
- eine aus dem Schaft und dem Handgriff bestehende erste Baugruppe und der Einsatz nach einer klinischen Wiederaufbereitung wiederverwendet werden kann;
- aufgrund der ersten und zweiten Befestigungsmittel am hintersten Drehmomentbegrenzungselement und am Einsatz eine zweite Baugruppe gebildet werden kann, welche lediglich in die erste Baugruppe eingeschoben und verriegelt werden muss;
- aufgrund der Austauschbarkeit der Drehmomentübertragungseinheit und des chirurgischen Werkzeugs anwendungsspezifisch unterschiedliche Werkzeuge und bezüglich des zu übertragenden Drehmoments unterschiedliche Drehmomentübertragungseinheiten mit derselben ersten Baugruppe verwendet werden können; und
- die Drehmomentübertragungseinheit kann als steriles Produkt geliefert werden oder aber auch (im Instrumentensieb) den wiederverwendbaren Komponenten der Vorrichtung beigelegt und im Autoklaven sterilisiert werden.

Weitere vorteilhafte Ausgestaltungen der Erfindung können wie folgt kommentiert werden:
In einer speziellen Ausführungsform der Vorrichtung umfasst der Einsatz eine Kammer zur Aufnahme von im Gebrauch der Vorrichtung abgetrennten Drehmomentbegrenzungselementen. Damit ist der Vorteil erreichbar, dass für einen Austausch der Drehmomentübertragungseinrichtung nur der Einsatz aus dem Handgriff herausgezogen werden muss. Die abgetrennten Drehmomentbegrenzungselemente können dann aus der Kammer entleert und eine neue ungebrauchte Drehmomentübertragungseinheit am Einsatz befestigt werden.

In einer weiteren Ausführungsform sind die am Einsatz angeordneten zweiten Befestigungsmittel in eine erste Position und in eine zweite Position bringbar, wobei in der ersten Position eine ungebrauchte Drehmomentübertragungseinheit lösbar am Einsatz fixierbar ist und in der zweiten Position der Einsatz axial und rotativ am Handgriff fixierbar ist. Für die Montage der Vorrichtung kann daher mit einer Hand die erste Baugruppe (Werkzeug, Schaft und Handgriff) gehalten werden, während mit der zweiten Hand die zweite Baugruppe (Einsatz und Drehmomentübertragungseinheit) gehalten und gleichzeitig die zweiten Befestigungsmittel bedient werden können. Die zweite Baugruppe kann somit mit einer Hand ohne Griffwechsel in die erste Baugruppe eingeführt und in dieser verriegelt werden. Ferner ergibt sich eine kompakte Bauweise der Vorrichtung.

In einer weiteren Ausführungsform umfassen die zweiten Befestigungsmittel einen Druckknopf und eine Feder, wobei der Druckknopf gegen die Federkraft der Feder in die erste Position bringbar ist und mittels der Federkraft der Feder in die zweite Position bringbar ist. Dadurch ist der Vorteil erreichbar, dass die zweiten Befestigungsmittel einmal zum Festhalten der Drehmomentübertragungseinheit bei der Montage und zum anderen nach dem Zusammenfügen für die Fixierung des Einsatzes im Handgriff dienen können.

In wiederum einer weiteren Ausführungsform ist das Werkzeug untrennbar mit dem Schaft verbunden und in der Kavität und an der Drehmomentübertragungseinheit sind komplementär ausgebildete Positioniermittel angeordnet, welche nur in einer bestimmten Kombination von Drehmomentübertragungseinheit und Schaft miteinander in Eingriff bringbar sind. Diese Konfiguration ergibt den Vorteil, dass z.B. nach dem Schlüssel/Schloss-Prinzip eine eindeutige unverwechselbare Zuordnung von Werkzeug und Drehmomentübertragungseinheit herstellbar ist.

In einer anderen Ausführungsform weisen die Drehmomentbegrenzungselemente orthogonal zur Längsachse eine nicht kreisrunde Querschnittsfläche auf. Vorzugsweise umfassen die Mittel zur drehfesten Verbindung des Einsatzes mit dem jeweils hintersten Drehmomentbegrenzungselement eine Durchgangsöffnung mit einer nicht kreisrunden Querschnittsfläche orthogonal zur Längsachse, welche zur Querschnittsfläche der Drehmomentbegrenzungselemente passgenau ist, so dass die Durchgangsöffnung mit dem jeweils hintersten Drehmomentbegrenzungselement formschlüssig in Eingriff bringbar ist. Ebenfalls vorzugsweise weist die Kavität im Schaft mindestens im Bereich des hinteren Endes des Schaftes eine nicht kreisrunde Querschnittsfläche auf, so dass die Kavität mindestens in diesem Bereich formschlüssig mit einem oder mehreren Drehmomentbegrenzungselementen in Eingriff bringbar ist. Dadurch kann die Drehmomentübertragung zwischen dem drehfest mit dem Handgriff verbundenen Einsatz und dem Schaft via die Drehmomentbegrenzungselemente auf eine einfache konstruktive Art und Weise hergestellt werden.

In einer weiteren Ausführungsform umfasst der Schaft einen Ratschenmechanismus, welcher in einer Rotationsrichtung um die Längsachse kein Drehmoment überträgt, und/oder ein Planetengetriebe zur Untersetzung des Drehmoments. Damit ist der Vorteil erreichbar, dass eine freie Drehung zum Ausholen beispielsweise beim Anziehen einer Schraube ermöglicht wird.

In einer weiteren Ausführungsform sind mindestens am Einsatz und am Handgriff RFID-Tags angebracht. Die Rückverfolgbarkeit der einzelnen Komponenten (für den Anwender untrennbaren Baugruppen) kann (neben den für gewöhnlich lesbaren Artikel- und Lotnummern etc.) mit RFID-Tags realisiert werden, um eine automatisierte Auslesung der Daten zu ermöglichen. Die Tags werden in den Baugruppen integriert, z.B. durch Kleben, Umspritzen, Klemmen, Schrauben oder einer sonstigen dem Stand der Technik entsprechenden Fügetechnik.

In einer anderen Ausführungsform umfasst die Vorrichtung zusätzlich einen T-Griff, welcher am Handgriff oder Einsatz lösbar befestigbar ist. Durch den T-Griff wird das Aufbringen von höheren Drehmomenten (in der Regel ab 6 Nm) ermöglicht. Beispielsweise kann der Handgriff direkt oder indirekt über den verbundenen Container dem T-Griff gekoppelt werden.

In einer anderen Ausführungsform umfasst die Ladeeinrichtung eine Druckfeder und ein Anpressteil, wobei das Anpressteil mittels Federkraft der Druckfeder gegen ein vorderes Ende der Drehmomentübertragungseinheit gedrückt wird.

In einer weiteren Ausführungsform umfasst der Schaft einen entlang der Längsachse verschiebbaren Markierungsring, welcher mit dem Anpressteil verbunden ist, wobei der Handgriff entlang der Längsachse voneinander beabstandete seitliche Öffnungen umfasst, so dass der Markierungsring je nach Position durch eine der seitlichen Öffnungen sichtbar ist. Der Markierungsring wird beim axialen Verschieben der Drehmomentübertragungseinheit zusammen mit dem Anpressteil verschoben, wenn ein nächstes Drehmomentbegrenzungselement mit den Mitteln zur drehfesten Verbindung des Einsatzes als hinterstes Drehmomentbegrenzungselement in Eingriff gebracht wird. Der Markierungsring und die seitlichen Öffnungen am Handgriff bilden somit eine Füllstandsanzeige für die an der Drehmomentbegrenzungseinheit noch vorhandenen Drehmomentbegrenzungselemente.

Vorzugsweise umfasst der Markierungsring ein lichtleitendes und/oder lumineszierendes Material. Damit wird eine bessere Ablesbarkeit der Position des Markierungsringes, d.h. der Füllstandsanzeige ermöglicht. Licht kann seitlich durch die weiteren benachbarten Öffnungen (hier Bohrungen) einfallen und durch leichte Vertiefungen oder rauere Stellen des Markierungsringes wieder austreten, um ein Leuchten in dem sonst dunklen Hintergrund zu erzeugen.

Gemäss einem weiteren Aspekt der Erfindung wird eine Kombination bereitgestellt, welche eine Vorrichtung wie oben dargestellt und eine elektronische Lesevorrichtung umfasst, mittels welcher die bei der Verwendung der Vorrichtung an der Drehmomentübertragungseinheit verbleibende Anzahl Drehmomentbegrenzungselemente detektierbar ist. Damit wird ein semiautomatische Auslesen und Dokumentieren der tatsächlichen Aktivierungen der Vorrichtung ermöglicht. Die Dokumentation kann von enormer Wichtigkeit sein. Hier können später Verifizierungen vorgenommen werden, wie zum Beispiel die Anzahl zu fixierender Schrauben mit den tatsächlich ausgelösten Aktivierungen oder eine reine Beweisführung, dass der Anzug der Implantatschrauben entsprechend der Vorgabe durchgeführt wurde. Entsprechend können Daten gesammelt und ausgewertet werden.

In einer anderen Ausführungsform umfasst die Lesevorrichtung Mittel zum Lesen von RFID-Tags. Hiermit können Informationen betreffend die verwendeten Baukomponenten zeitgleich mit dem Auslesen der Aktivierungen eingeholt werden.

In einer anderen Ausführungsform umfasst die elektronische Lesevorrichtung eine sterilisierbare Schale, welche eine Vertiefung zur Aufnahme des Handgriffs aufweist.

Vorzugsweise ist die Vertiefung für eine passgenaue Aufnahme des Handgriffs ausgebildet.

In einer weiteren Ausführungsform umfasst die Lesevorrichtung Abstandsmesssensoren, mittels welcher die Position des Markierungsrings entlang der Längsachse detektierbar ist und welche vorzugsweise als optische, infrarot- oder akustische Sensoren ausgebildet sind.

In einer weiteren Ausführungsform ist die Schale aus einem Material hergestellt, welches durchlässig für Infrarotstrahlung oder Ultraschall ist.

In wiederum einer weiteren Ausführungsform weisen die Abstandsmesssensoren einen Sensorfokus auf, welcher in einem Abstand von den Abstandsmesssensoren liegt, der bei einer in die Schale eingelegten Vorrichtung in den Bereich des Markierungsrings fällt.

In einer anderen Ausführungsform entspricht die Anzahl der Abstandsmesssensoren der Lesevorrichtung der Anzahl Drehmomentbegrenzungselemente der Drehmomentübertragungseinheit.

In einer anderen Ausführungsform weisen die Abstandsmesssensoren einen Abstand zueinander auf, welcher der in Richtung der Längsachse gemessenen Länge der Drehmomentbegrenzungselemente entspricht. Damit ist der Vorteil erreichbar, dass die Anzahl der Aktivierungen der Vorrichtung via die Anzahl der an der Drehmomentübertragungseinheit verbleibenden Drehmomentbegrenzungselemente ermittelt werden kann.

In einer anderen Ausführungsform sind die Abstandsmesssensoren auf einer Grundplatte angeordnet und elektrisch mit einer elektronischen Schnittstelle zu einem externen Computer verbunden.

In einer weiteren Ausführungsform ermöglicht die Schnittstelle eine drahtlose Datenübertragung zu einem externen Computer.

In einer weiteren Ausführungsform sind die Abstandsmesssensoren und die elektronische Schnittstelle in der Schale durch Verkleben, Beschichten oder Vergiessen mechanisch, elektromagnetisch und vorzugsweise thermisch verkapselt.

In wiederum einer weiteren Ausführungsform umfasst die Schale eine interne Energiequelle, vorzugsweise mindestens einen Akkumulator.

In einer anderen Ausführungsform umfassen die Mittel zum Auslesen der RFID-Tags eine Antenne.

In einer anderen Ausführungsform umfasst die Schale optische und/oder akustische Anzeigeelemente. Dies ermöglicht eine Anzeige z.B. zur Quittierung einer Situation wie erfolgreiche Ablesung, Auftreten eines Fehlers etc.

In einer weiteren Ausführungsform umfasst die Kombination zusätzlich einen Computer, welcher dazu geeignet programmiert ist, via die Abstandsmesssensoren automatisch oder semiautomatisch zu erfassen, wie viele Drehmomentbegrenzungselemente im Gebrauch der Vorrichtung von einer eingesetzten Drehmmomentübertragungseinheit abgetrennt wurden. Dadurch ist der Vorteil erreichbar, dass ein Auslesen und Dokumentieren der tatsächlichen Aktivierungen der Vorrichtung ermöglicht wird.

Gemäss einem anderen Aspekt der Erfindung wird ein Kit bereitgestellt, welcher eine Vorrichtung wie oben dargestellt und eine Mehrzahl unterschiedlicher Sets umfasst, wobei jedes Set folgendes umfasst:
- ein untrennbar mit einem Schaft verbundenes Werkzeug, welches an einem freie Ende Mittel zur Übertragung eines Drehmoments auf ein chirurgisches Implantat oder Werkzeug aufweist; und
- eine Drehmomentübertragungseinheit, deren Sollbruchstellen das zu übertragende Drehmoment auf einen Wert begrenzen, welcher den Mitteln zur Übertragung eines Drehmoments am freien Ende des Werkzeug angepasst ist und/oder für eine beabsichtigte Verwendung der Vorrichtung wählbar ist; wobei in der Kavität des Schaftes und an der Drehmomentübertragungseinheit für jedes Set unterschiedliche, komplementär ausgebildete Positioniermittel angeordnet sind, welche nur in dieser Kombination von Drehmomentübertragungseinheit und Schaft miteinander in Eingriff bringbar sind.

In einer anderen Ausführungsform sind die Mittel zur Übertragung eines Drehmoments am freien Ende des Werkzeugs als Sechskant, Torx oder Sonderform ausgebildet.

Gemäss einem weiteren Aspekt der Erfindung wird ein Verfahren zur Montage der oben dargestellten Vorrichtung bereitgestellt, welches folgende Schritte aufweist: a) Einsetzen des Schaftes zusammen mit dem am vorderen Ende des Schaftes angeordneten Werkzeug in den Handgriff, wodurch eine erste Baugruppe gebildet wird; b) Verbinden des Einsatzes mit einer ungebrauchten Drehmomentübertragungseinheit, wobei die ersten Befestigungsmittel am hintersten Drehmomentbegrenzungselement der ungebrauchten Drehmomentübertragungseinheit mit den zweiten Befestigungsmitteln am Einsatz in Eingriff gebracht werden und die Mittel zur drehfesten Verbindung des Einsatzes mit dem jeweils hintersten Drehmomentbegrenzungselement in Eingriff gebracht werden, wodurch eine zweite Baugruppe gebildet wird; c) Einsetzen der zweiten Baugruppe in die erste Baugruppe; und d) Verriegeln des Einsatzes im Handgriff.

In einer weiteren Ausführungsform des Verfahrens werden in Schritt b) die zweiten Befestigungsmittel am Einsatz durch Drücken eines Druckknopfes gegen die Federkraft einer Feder in eine erste Position gebracht, in welcher die ungebrauchte Drehmomentübertragungseinheit lösbar am Einsatz fixiert ist.

In einer anderen Ausführungsform des Verfahrens wird in Schritt c) der Druckknopf gedrückt gehalten, so dass beim Einsetzen der zweiten Baugruppe in die erste Baugruppe die Drehmomentübertragungseinheit am Einsatz fixiert ist.

In einer anderen Ausführungsform wird in Schritt d) der Druckknopf losgelassen, so dass die zweiten Befestigungsmittel mittels der Federkraft der Feder in die zweite Position gebracht werden, in welcher der Einsatz axial und rotativ am Handgriff fixiert ist und die Fixation der ungebrauchten Drehmomentübertragungseinheit am Einsatz gelöst wird.

Die Erfindung und Weiterbildungen der Erfindung werden im Folgenden anhand der teilweise schematischen Darstellungen mehrerer Ausführungsbeispiele noch näher erläutert.

Es zeigen:
Fig. 1 einen Längsschnitt durch eine Ausführungsform der erfindungsgemässen Vorrichtung;
Fig. 2 einen Längsschnitt durch die in Fig. 1 dargestellte Ausführungsform der erfindungsgemässen Vorrichtung, wobei die aus Drehmomentübertragungseinheit und Einsatz bestehende Baugruppe demontiert ist;
Fig. 3 eine perspektivische Ansicht der Drehmomentübertragungseinheit und des hinteren Endes des Schaftes einer weiteren Ausführungsform der erfindungsgemässen Vorrichtung;
Fig. 4 eine perspektivische Ansicht der Drehmomentübertragungseinheit und des Einsatzes einer anderen Ausführungsform der erfindungsgemässen Vorrichtung;
Fig. 5 eine perspektivische Ansicht des mit einem Werkzeug verbundenen Schaftes und der mit dem Einsatz verbundenen Drehmomentübertragungseinheit gemäss der in Fig. 4 dargestellten Ausführungsform der erfindungsgemässen Vorrichtung vor der Montage der ersten und zweiten Baugruppe;

Fig. 6 eine perspektivische Ansicht des mit einem Werkzeug verbundenen Schaftes und der mit dem Einsatz verbundenen Drehmomentübertragungseinheit gemäss der in Fig. 4 dargestellten Ausführungsform der erfindungsgemässen Vorrichtung nach der Montage der ersten und zweiten Baugruppe; und

Fig. 7 einen Längsschnitt durch eine Ausführungsform der erfindungsgemässen Kombination.

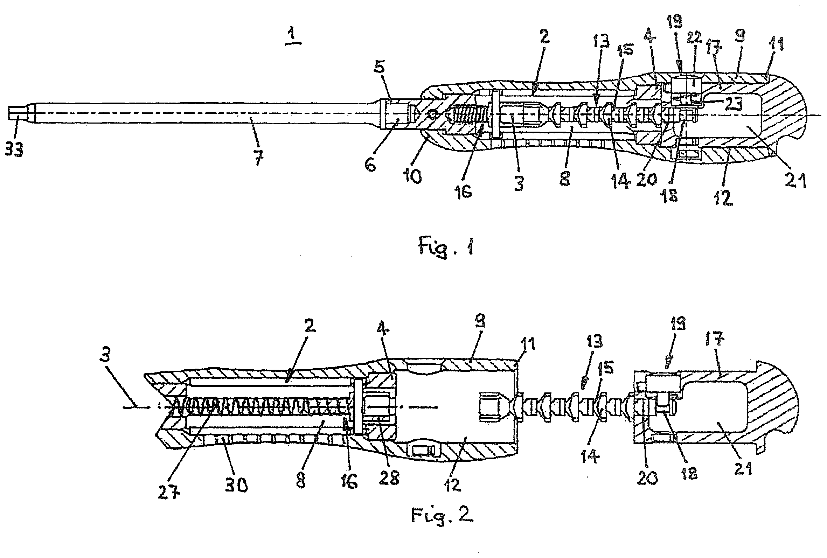

Die in den Fig. 1 - 3 dargestellte Ausführungsform der erfindungsgemässen Vorrichtung 1 umfasst im Wesentlichen einen longitudinalen Schaft 2 mit einer Längsachse 3, an dessen vorderen Ende 5 ein chirurgisches Werkzeug 7 befestigt ist, einen Handgriff 9 mit einem Hohlraum 12, in welchem der Schaft 2 gelagert ist, eine Drehmomentübertragungseinheit 13 mit einer Mehrzahl von Drehmomentbegrenzungselementen 14, welche entlang der Längsachse 3 hintereinander angeordnet und jeweils über eine Sollbruchstelle 15 miteinander verbunden sind, und einen Einsatz 17, welcher im montierten Zustand der Vorrichtung 1 in den Hohlraum 12 des Handgriffs 9 eingeführt und am Handgriff 9 lösbar axial und rotativ fixiert ist. Der Einsatz 17 umfasst Mittel 20 zur drehfesten Verbindung mit dem jeweils hintersten Drehmomentbegrenzungselement 14.

Ferner ist die Drehmomentübertragungseinheit 13 - im montierten Zustand der Vorrichtung 1 - drehfest aber koaxial zur Längsachse 3 verschiebbar in der Kavität 8 des Schaftes 2 angeordnet, so dass ein hinterstes Drehmomentbegrenzungselement 14 über das hinter Ende 4 des Schaftes 2 hinausragt und via den Einsatz 17 drehfest mit dem Handgriff 9 verbunden ist. Bei einem Überschreiten eines vorgegebenen Drehmoments wird das hinterste Drehmomentbegrenzungselement 14 an der Sollbruchstelle 15 abgetrennt.

Der Schaft 2 umfasst ferner eine Ladeeinrichtung 16, mittels welcher die Drehmomentübertragungseinheit 13 relativ zur Längsachse 3 des Schaftes 2 axial gegen das hintere Ende 4 des Schafts 2 verschiebbar ist, so dass nach einem Abtrennen des hintersten Drehmomentbegrenzungselements 14 das axial angrenzende Drehmomentbegrenzungselement 14 über das hintere Ende 4 des Schaftes 2 hinausragt und wiederum als hinterstes Drehmomentbegrenzungselement 14 via den Einsatz 17 drehfest mit dem Handgriff 9 verbunden ist. Wird beispielsweise eine Knochenschraube mittels der Vorrichtung 1 in einen Knochen eingedreht, so erfolgt beim Überschreiten eines gewünschten Anzugsdrehmoments eine mechanische Abtrennung des auf der Arbeitsachse liegenden Drehmomentbegrenzungselements 14.

Mit Ausnahme der Drehmomentübertragungseinheit 13 mit den Drehmomentbegrenzungselementen 14 lassen sich die Bauteile der Vorrichtung 1 klinisch aufbereiten und wieder verwenden. Die Drehmomentbegrenzungselemente 14 werden nach der Zerstörung der Drehmomentübertragungseinheit 13 entsorgt. Dazu umfasst der Einsatz 17 eine Kammer 21, welche zur Aufnahme der im Gebrauch der Vorrichtung 1 abgetrennten Drehmomentbegrenzungselemente 14, so dass der Einsatz 17 eine separate Auffangeinheit bildet.

Für einen Austausch der Drehmomentübertragungseinheit 13 muss daher nur der Einsatz 17 aus dem Handgriff 9 herausgezogen werden. Die abgetrennten Drehmomentbegrenzungselemente 14 können dann aus der Kammer 21 entleert und eine neue ungebrauchte Drehmomentübertragungseinheit 13 am Einsatz 17 befestigt werden.

Zudem umfassen das hinterste Drehmomentbegrenzungselement 14 der ungebrauchten Drehmomentübertragungseinheit 13 erste Befestigungsmittel 18 und der Einsatz 17 zweite Befestigungsmittel 19, welche zur Befestigung der ungebrauchten Drehmomentübertragungseinheit 13 am Einsatz 17 miteinander in Eingriff bringbar sind. Dazu sind die am Einsatz 17 angeordneten zweiten Befestigungsmittel 19 in eine erste Position und in eine zweite Position bringbar, wobei in der ersten Position eine ungebrauchte Drehmomentübertragungseinheit 13 lösbar am Einsatz 17 fixierbar ist und in der zweiten Position der Einsatz 17 axial und rotativ am Handgriff 9 fixierbar ist.

Bei der Montage der Vorrichtung 1 wird zuerst der Schaft 2 in den Handgriff 9 eingesetzt. Ein am Schaft 2 angebrachtes Kugeldruckstück 34 dient als Verliersicherung, mittels welcher der Schaft 2 lösbar am Handgriff 9 gesichert ist.

Die am Einsatz 17 angeordneten zweiten Befestigungsmittel 19 umfassen einen Druckknopf 22 und eine Feder 23, wobei der Druckknopf 22 bei der Montage zuerst gegen die Federkraft der Feder 23 in die erste Position gebracht wird, wo das vordere Ende des Druckknopfs 22 in die als Vertiefung am hinteren Ende der ungebrauchten Drehmomentübertragungseinheit 13 ausgebildeten ersten Befestigungsmittel 19 eingreift. Der Druckknopf 22 wird zum Arretieren der Drehmomentübertragungseinheit 13 am Einsatz 17 während der Montage gedrückt gehalten bis die Drehmomentübertragungseinheit 13 in die Kavität 8 im Schaft 2 und der Einsatz 17 in Hohlraum 12 im Handgriff 9 eingeführt sind.

Danach wird der Druckknopf 22 losgelassen und der Einsatz 17 leicht um die Längsachse 3 gedreht, so dass der Druckknopf 22 mittels der Federkraft der Feder 23 in die zweite Position gebracht wird und der Einsatz 17 im Handgriff 9 axial und rotativ fixiert wird. Mithilfe des Einsatzes 17, insbesondere des Druckknopfs 22 kann daher die gesamte Vorrichtung 1 verriegelt und entriegelt werden.

Die Vorrichtung 1 ist somit so konzipiert, dass durch den Schaft 2, den Handgriff 9 und das am Schaft 2 befestigte Werkzeug 7 eine erste Baugruppe gebildet wird, während der Einsatz 17 und die während der Montage am Einsatz 17 befestigte ungebrauchte Drehmomentübertragungseinheit 13 eine zweite Baugruppe bilden.

Damit kann der Anwender die Komponenten der Vorrichtung 1 mit wenigen Handgriffen demontieren und entsprechend den allgemeingültigen klinischen Wiederaufbereitungsverfahren erneut nutzen. Lediglich die Drehmomentübertragungseinheit 13 wird neu eingesetzt. Die Montage kann intraoperativ durchgeführt werden.

Der Handgriff 9 umfasst ein erstes Ende 10, ein zweites Ende 11 und einem den Handgriff 9 entlang der Längsachse 3 vom ersten bis zum zweiten Ende 10, 11 durchdringenden Hohlraum 12, in welchem der Schaft 2 vor der Montage und Verriegelung der ersten und zweiten Baugruppe um die Längsachse 3 rotierbar gelagert ist.

Die Drehmomentbegrenzungselemente 14 weisen orthogonal zur Längsachse 3 eine nicht kreisrunde Querschnittsfläche auf, welche beispielhaft, und nicht einschränkend, durch eine mittels zwei parallelen einander gegenüberliegenden Seiten beschnittene Kreisfläche gebildet wird. Ferner sind die Drehmomentbegrenzungselemente 14 so angeordnet, dass je zwei benachbarte Drehmomentbegrenzungselemente 14 um 90° um die Längsachse 3 gedreht sind, so dass nach einem Abtrennen des hintersten Drehmomentbegrenzungselements 14 durch eine Drehung des Handgriffs 9 um 90° die Drehmomentübertragungseinheit 13 mittels der Ladeeinrichtung 16 relativ zur Längsachse 3 des Schaftes 2 axial gegen das hintere Ende 4 des Schafts 2 verschoben wird bis das axial angrenzende Drehmomentbegrenzungselement 14 über das hintere Ende 4 des Schaftes 2 hinausragt und wiederum als hinterstes Drehmomentbegrenzungselement 14 via den Einsatz 17 drehfest mit dem Handgriff 9 verbunden ist.

Zur Drehmomentübertragung zwischen dem drehfest mit dem Handgriff 9 verbundenen Einsatz 17 und dem Schaft 2 via die Drehmomentbegrenzungselemente 14 umfassen die Mittel 20 zur drehfesten Verbindung des Einsatzes 17 mit dem jeweils hintersten Drehmomentbegrenzungselement 14 eine Durchgangsöffnung 25 mit einer nicht kreisrunden, zur Querschnittsfläche der Drehmomentbegrenzungselemente 14 komplementären Querschnittsfläche orthogonal zur Längsachse 3, so dass die Durchgangsöffnung 25 mit dem jeweils hintersten Drehmomentbegrenzungselement 14 formschlüssig in Eingriff bringbar ist. Ebenfalls weist die Kavität 8 im Schaft 2 beispielhaft im Bereich des hinteren Endes 4 des Schaftes 2 eine nicht kreisrunde, zur Querschnittsfläche der Drehmomentbegrenzungselemente 14 komplementäre Querschnittsfläche auf, so dass die Kavität 8 und damit der Schaft 2 in diesem Bereich formschlüssig mit dem an das jeweils hinterste Drehmomentbegrenzungselement 14 angrenzenden Drehmomentbegrenzungselement 14 in Eingriff bringbar ist.

Die Ladeeinrichtung 16 umfasst eine Druckfeder 27 und ein Anpressteil 28, welches mittels Federkraft der Druckfeder 27 gegen das vordere Ende der Drehmomentübertragungseinheit 13 gedrückt wird. Die Drehmomentbegrenzungselemente 14 und die Kavität 8 erweitern sich im Durchmesser komplementär gegen das hintere Ende 4 des Schaftes 2. Dadurch wird die mit steigender Ausdehnung der Druckfeder 27 abnehmende Anpresskraft des an das jeweils hinterste Drehmomentbegrenzungselement 14 angrenzenden Drehmomentbegrenzungselements 14 an die Stirnfläche des Einsatzes 17 kompensiert. Die theoretische Reibkraft zwischen der Drehmomentübertragungseinheit 13 und dem Einsatz 17, welche ohne diese Kompensation ebenfalls abnehmen würde, wird durch die sich konisch erweiternden Durchmesser so angepasst, dass am Arbeitsende wieder das gewünschte Nenndrehmoment (mit minimaler fertigungsbedingter Abweichung) anliegt.

Die Kavität 8 im Schaft 2 ist zur Längsachse 3 koaxial ausgebildet und am hinteren Ende 4 des Schafts 2 offen. Ferner sind in einem mittleren Längsabschnitt des Schaftes 2 Längsschlitze 35 angeordnet. Der Schaft 2 umfasst einen entlang der Längsachse 3 verschiebbaren Markierungsring 29, welcher mit dem Anpressteil 28 der Ladeeinrichtung 16 verbunden ist. Zudem weist der Handgriff 9 entlang der Längsachse 3 voneinander beabstandete seitliche Öffnungen 30 auf, so dass der Markierungsring 29 je nach Position durch eine der seitlichen Öffnungen 30 sichtbar ist.

Der Markierungsring 29 wird beim axialen Verschieben der Drehmomentübertragungseinheit 13 zusammen mit dem Anpressteil 28 verschoben, wenn ein nächstes Drehmomentbegrenzungselement 14 mit den Mitteln 20 zur drehfesten Verbindung des Einsatzes 17 mit dem jeweils hintersten Drehmomentbegrenzungselement 14 in Eingriff gebracht wird. Der Markierungsring 29 ist durch eine der seitlichen Öffnungen 30 am Handgriff 9 sichtbar, so dass durch den Markierungsring 29 eine Füllstandsanzeige für die an der Drehmomentbegrenzungseinheit 13 noch vorhandenen Drehmomentbegrenzungselemente 14.

Der Markierungsring 29 umfasst beispielhaft, und nicht einschränkend ein lichtleitendes und/oder lumineszierendes Material, so dass eine bessere Ablesbarkeit der Position des Markierungsringes 29 ermöglicht wird.

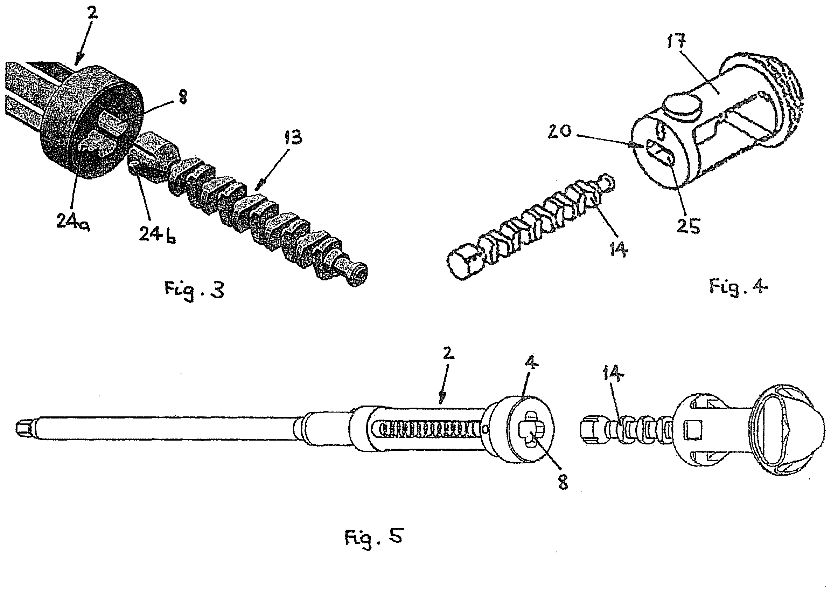

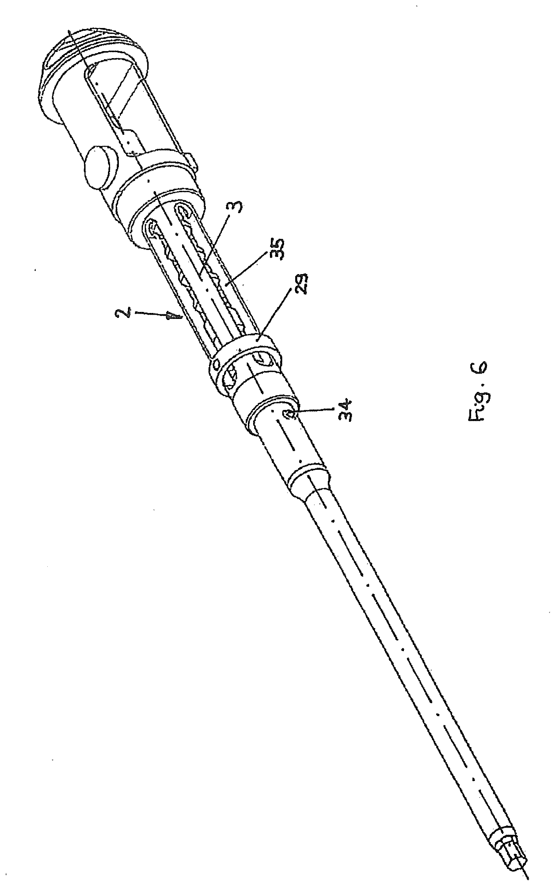

In den Fig. 4 - 6 ist eine alternative Ausführungsform der erfindungsgemässen Vorrichtung 1 dargestellt, welche sich von der in den Fig. 1 - 3 gezeigten Ausführungsform nur darin unterscheidet, dass das Werkzeug 7 untrennbar mit dem Schaft 2 verbunden ist und zudem in der Kavität 8 und an der Drehmomentübertragungseinheit 13 komplementär ausgebildete Positioniermittel 24a, 24b angeordnet sind, welche nur in einer bestimmten Kombination von Drehmomentübertragungseinheit 13 und Schaft 2 miteinander in Eingriff bringbar sind.

Die Positioniermittel 24a, 24b sind beispielhaft, und nicht einschränkend, durch einen Stift am vorderen Ende der Drehmomentübertragungseinheit 13 und durch eine Aussparung in der Kavität 8 des Schaftes 2 realisiert. Es sind jedoch beliebige, dem Fachmann bekannte Kombinationen denkbar.

Dadurch hat der Anwender verschiedene Drehmomentbegrenzungseinheiten 13 und verschiedene Werkzeuge 7 (z.B. Torx, Sechskant, Sonderformen etc.) zur Verfügung und kann somit anwendungsspezifisch verschiedene Drehmomente übertragen. Durch die Positioniermittel 24a, 24b ist eine Verwechslung bzw. Mischung untereinander konstruktiv ausgeschlossen.

In einer weiteren alternativen Ausführungsform umfasst der Schaft 2 einen Ratschenmechanismus (nicht gezeichnet), welcher in einer Rotationsrichtung um die Längsachse 3 kein Drehmoment überträgt, so dass die Verwendung, bzw. das Anziehen von Schrauben mithilfe der erfindungsgemässen Vorrichtung 1 vereinfacht wird, und/oder ein Planetengetriebe zur Untersetzung des Drehmoments. Der Schaft 2 ist dazu mit einem einseitigen Ratschenmechanismus versehen (z.B. Linksdrehung freilaufend und Rechtsdrehung sperrend), welcher beispielsweise für den Fachmann in bekannter Weise als Kupplung mit Stirnverzahnung ausgeführt sein kann.

In einer anderen Ausführungsform umfasst die Vorrichtung 1 zusätzlich einen T-Griff (nicht gezeichnet), welcher am Handgriff 9 oder am Einsatz 17 lösbar befestigt werden kann, so dass auch höhere Drehmomente aufgebracht werden können. Der T-Griff kann über eine dem Fachmann bekannte Schnittstelle mit dem Handgriff 9, respektive dem Einsatz 17 gekoppelt werden. Beispielsweise kann der T-Griff über einen Druckknopf mit Schaft eine Kugel aus einer im Einsatz 17 befindlichen Vertiefung lösen. Bei gelöstem Druckknopf drückt eine Feder eine zu einem Keil geformte Aussparung des Schaftes des Druckknopfes gegen die Kugel und drückt diese in die Vertiefung des Einsatzes 17. Diese Bauart unterliegt der Selbsthemmung d.h. der T-Griff kann sich nur aktiv durch Drücken des Druckknopfes lösen. Der Schaft des Druckknopfes kann ausserdem derart geformt sein, dass er sich bei vollständigem Herunterdrücken von der Unterkante der Aussparung im Einsatz 17 abstützt und somit ein Entnehmen des T-Griffes erleichtert. Die Kugel fällt dabei in eine neutrale Position zurück und gibt den Schaft des Druckknopfs frei.

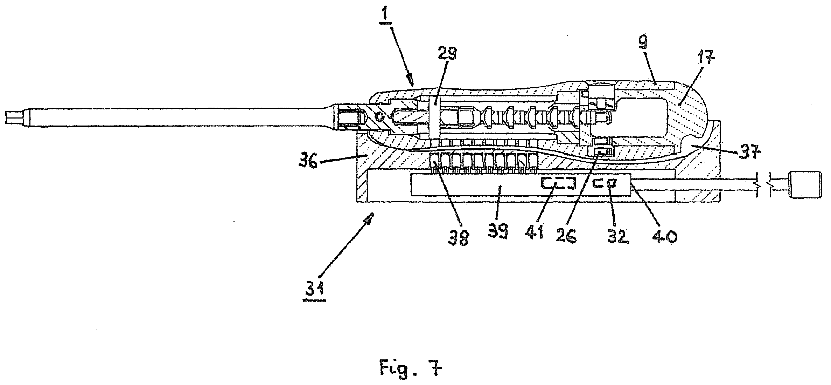

In einer weiteren Ausführungsform der erfindungsgemässen Vorrichtung 1 (Fig. 7) sind am Einsatz 17 und am Handgriff 9 je ein RFID-Tag 26 angebracht, um eine automatisierte Datenerfassung und Rückverfolgbarkeit, insbesondere bezüglich die einzelnen Komponenten der Vorrichtung 1 zu ermöglichen. Die RFID-Tags 26 werden durch Kleben, Umspritzen, Klemmen, Schrauben oder sonstigen dem Fachmann bekannten Fügetechniken in den Baugruppen integriert.

Fig. 7 zeigt eine Ausführungsform der erfindungsgemässen Kombination, welche neben einer der oben dargestellten Ausführungsformen der erfindungsgemässen Vorrichtung 1 eine elektronische Lesevorrichtung 31 umfasst. Die elektronische Lesevorrichtung 31 umfasst eine sterilisierbare Schale 36, welche eine Vertiefung 37 zur passgenauen Aufnahme des Handgriffs 9 aufweist. Um die Elektronik der Lesevorrichtung 31 zu schützen, sind an der Schale 36 keine Bohrungen, Öffnungen oder andere Hinterschnitte angebracht, so dass die Oberfläche der Schale 36 sehr glatt ist.

Die Lesevorrichtung 31 umfasst Abstandsmesssensoren 38, mittels welcher die Position des Markierungsrings 29 entlang der Längsachse 3 detektierbar ist und welche beispielhaft als optische, infrarot- oder akustische Sensoren ausgebildet sind. Zudem ist die Schale 36 aus einem Material hergestellt, welches durchlässig für Infrarotstrahlung und/oder Ultraschall ist. Alternativ können die Abstandsmesssensoren 38 einen Sensorfokus aufweisen, welcher in einem Abstand von den Abstandsmesssensoren 38 liegt, der bei einer in die Schale 36 eingelegten Vorrichtung 1 in den Bereich des Markierungsrings 29 fällt. Die Anzahl der Abstandsmesssensoren 38 der Lesevorrichtung 31 entspricht dabei der Anzahl Drehmomentbegrenzungselemente 14 der Drehmomentübertragungseinheit 13, wobei die Abstandsmesssensoren 38 einen Abstand zueinander aufweisen, welcher der in Richtung der Längsachse 3 gemessenen Länge der Drehmomentbegrenzungselemente 14 entspricht.

Die Abstandsmesssensoren 38 sind auf einer Grundplatte 39 (z.B. elektronische Leiterplatte) angeordnet und sind elektrisch mit einer elektronischen Schnittstelle 40 verbunden, welche eine elektronische Verbindung (mittels Kabeln oder drahtlos) zu einem externen Computer ermöglicht.

Die Schale 36 ist so konfiguriert, dass die Abstandsmesssensoren 38 und die elektronische Schnittstelle 40 durch Verkleben, Beschichten oder Vergiessen mechanisch, elektromagnetisch und vorzugsweise thermisch verkapselt sind.

Ferner umfasst die Schale 36 beispielhaft, und nicht einschränkend, eine interne Energiequelle 41, vorzugsweise eine oder mehrere Batterien oder Akkumulatoren, welche beispielsweise mittels Induktion kontaktlos geladen werden können.

Zusätzlich umfasst die Lesevorrichtung 31 Mittel 32 zum Lesen von RFID-Tags 26, wobei die Mittel 32 beispielhaft eine Antenne zum Auslesen der RFID-Tags 26 umfassen. Hiermit können entsprechende Informationen der Baukomponenten erfasst und zeitgleich mit dem Auslesen der Anzahl Aktivierungen übertragen werden.

In einer weiteren Ausführungsform kann die Schale 36 optische und/oder akustische Anzeigeelemente (z.B. Leuchtdioden oder akustische Signalgeber) umfassen, welche beispielsweise eine Quittierung einer Situation wie erfolgreiche Ablesung, Auftreten eines Fehlers etc. anzeigen.

Die Kombination kann zudem einen Computer umfassen, welcher dazu geeignet programmiert ist, via die Abstandsmesssensoren 38 automatisch oder semiautomatisch zu erfassen, wie viele Drehmomentbegrenzungselemente 14 im Gebrauch der Vorrichtung 1 von einer eingesetzten Drehmmomentübertragungseinheit 13 abgetrennt wurden.

Die Vorrichtung 1 wird vom Anwender zum Auslesen (vor dem ersten Gebrauch, nach einem Wechsel der Drehmomentübertragungseinheit 13 oder nach Beendigung der OP) in die sterile passgenaue Schale 36 gelegt. Die Schale 36 ist mit einem sterilen Kabel mit Stecker mit einem weiteren nicht sterilen Kabel mit Stecker mit einem Computer, Handy oder handheld-Device verbunden, welche über entsprechende Software verfügen (z.B. über eine Standard-Schnittstelle wie USB, RS232 etc.). Alternativ wird die Verbindung kontaktlos über Funk, Bluetooth, Infrarotoptik oder ähnliche bekannte Methoden hergestellt. Die Schale 36 kann mit den klinischen Standard-Methoden gereinigt, sterilisiert und wiederaufbereitet werden.

Detektiert wird der Markierungsring 29 in der Vorrichtung 1. Seine Position gibt Aufschluss über die Anzahl der Auslösungen, welche später durch die Software ausgewertet werden kann. Zum Detektieren dienen die Abstandsmesssensoren 38, welche das Material bzw. messtechnisch nur das grobe Vorhandensein (digitale Situation, also 1 oder 0) des Markierungsringes 29 erfassen. Weitere Materialien sind entsprechend nicht detektierbar oder die Sensoren sind derart adaptiert, dass der Sensorfokus in dem bekannten Abstand zum Markierungsring 38 liegt.

Diese Erfindung ist nicht einfach auf die oben erwähnten, besonders bevorzugten Ausführungsformen beschränkt, sondern wird durch die nachstehend aufgeführten Ansprüche definiert.

## Patentansprüche

1. Vorrichtung (1) zur Übertragung eines vorgegebenen Drehmoments auf ein chirurgisches Werkzeug, wobei die Vorrichtung (1) umfasst:
A) einen longitudinalen Schaft (2) mit einer Längsachse (3), einem hinteren Ende (4) und einem vorderen Ende (5), wobei am vorderen Ende (5) ein chirurgisches Werkzeug (7) befestigt oder befestigbar ist und der Schaft (2) eine zur Längsachse (3) koaxiale Kavität (8) umfasst, welche am hinteren Ende (4) des Schafts (2) offen ist;
B) einen Handgriff (9) mit einem ersten Ende (10), einem zweiten Ende (11) und einem den Handgriff (9) entlang der Längsachse (3) vom ersten bis zum zweiten Ende (10, 11) durchdringenden Hohlraum (12), wobei der Schaft (2) mindestens auf einem Teil seiner Länge und um die Längsachse (3) rotierbar im Hohlraum (12) des Handgriffs (9) gelagert ist;
C) eine Drehmomentübertragungseinheit (13) mit einer Mehrzahl von Drehmomentbegrenzungselementen (14), welche entlang der Längsachse (3) hintereinander angeordnet und jeweils über eine Sollbruchstelle (15) miteinander verbunden sind, wobei die Drehmomentübertragungseinheit (13) drehfest aber koaxial zur Längsachse (3) verschiebbar in der Kavität (8) des Schaftes (2) angeordnet ist, so dass ein hinterstes Drehmomentbegrenzungselement (14) über das hinter Ende (4) des Schaftes (2) hinausragt und drehfest mit dem Handgriff (9) verbunden ist, wobei bei einem Überschreiten eines vorgegebenen Drehmoments das hinterste Drehmomentbegrenzungselement (14) an der Sollbruchstelle (15) abgetrennt wird; und wobei
D) der Schaft (2) eine Ladeeinrichtung (16) umfasst, mittels welcher die Drehmomentübertragungseinheit (13) relativ zur Längsachse (3) axial gegen das hintere Ende (4) des Schafts (2) verschiebbar ist, so dass nach einem Abtrennen des hintersten Drehmomentbegrenzungselements (14) das axial angrenzende Drehmomentbegrenzungselement (14) über das hintere Ende (4) des Schaftes (2) hinausragt und wiederum als hinterstes Drehmomentbegrenzungselement (14) drehfest mit dem Handgriff (9) verbunden ist,
**dadurch gekennzeichnet, dass**
E) die Vorrichtung (1) einen Einsatz (17) umfasst, welcher im montierten Zustand der Vorrichtung (1) in den Hohlraum (12) des Handgriffs (9) eingeführt und am Handgriff (9) lösbar axial und rotativ fixiert ist, wobei
F) das hinterste Drehmomentbegrenzungselement (14) der ungebrauchten Drehmomentübertragungseinheit (13) erste Befestigungsmittel (18) und der Einsatz (17) zweite Befestigungsmittel (19) umfassen, welche zur Befestigung der ungebrauchten Drehmomentübertragungseinheit (13) am Einsatz (17) miteinander in Eingriff bringbar sind; und
G) der Einsatz (17) Mittel (20) zur drehfesten Verbindung mit dem jeweils hintersten Drehmomentbegrenzungselement (14) umfasst.

2. Vorrichtung nach Anspruch 1, **dadurch gekennzeichnet, dass** der Einsatz (17) eine Kammer (21) zur Aufnahme von im Gebrauch der Vorrichtung (1) abgetrennten Drehmomentbegrenzungselementen (14) umfasst.

3. Vorrichtung nach Anspruch 1 oder, 2 **dadurch gekennzeichnet, dass** die am Einsatz (17) angeordneten zweiten Befestigungsmittel (19) in eine erste Position und in eine zweite Position bringbar sind, wobei in der ersten Position eine ungebrauchte Drehmomentübertragungseinheit (13) lösbar am Einsatz (17) fixierbar ist und in der zweiten Position der Einsatz (17) axial und rotativ am Handgriff (9) fixierbar ist.

4. Vorrichtung nach einem der Ansprüche 1 bis 3, **dadurch gekennzeichnet, dass** die zweiten Befestigungsmittel (19) einen Druckknopf (22) und eine Feder (23) umfassen, und dass der Druckknopf (22) gegen die Federkraft der Feder (23) in die erste Position bringbar ist und mittels der Federkraft der Feder (23) in die zweite Position bringbar ist.

5. Vorrichtung nach einem der Ansprüche 1 bis 4, **dadurch gekennzeichnet, dass** das Werkzeug (7) untrennbar mit dem Schaft (2) verbunden ist und in der Kavität (8) und an der Drehmomentübertragungseinheit (13) komplementär ausgebildete Positioniermittel (24a, 24b) angeordnet sind, welche nur in einer bestimmten Kombination von Drehmomentübertragungseinheit (13) und Schaft (2) miteinander in Eingriff bringbar sind.

6. Vorrichtung nach einem der Ansprüche 1 bis 5, **dadurch gekennzeichnet, dass** die Drehmomentbegrenzungselemente (14) orthogonal zur Längsachse (3) eine nicht kreisrunde Querschnittsfläche aufweisen, wobei die Mittel (20) zur drehfesten Verbindung des Einsatzes (17) mit dem jeweils hintersten Drehmomentbegrenzungselement (14) eine Durchgangsöffnung (25) mit einer nicht kreisrunden Querschnittsfläche orthogonal zur Längsachse (3) umfassen, welche zur Querschnittsfläche der Drehmomentbegrenzungselemente (14) passgenau ist, so dass die Durchgangsöffnung (25) mit dem jeweils hintersten Drehmomentbegrenzungselement (14) formschlüssig in Eingriff bringbar ist.

7. Vorrichtung nach Anspruch 6, **dadurch gekennzeichnet, dass** die Kavität (8) im Schaft (2) mindestens im Bereich des hinteren Endes (4) des Schaftes (2) eine nicht kreisrunde Querschnittsfläche aufweist, so dass die Kavität (8) mindestens in diesem Bereich formschlüssig mit einem oder mehreren Drehmomentbegrenzungselementen (14) in Eingriff bringbar ist.

8. Vorrichtung nach einem der Ansprüche 1 bis 7, **dadurch gekennzeichnet, dass** der Schaft (2) einen Ratschenmechanismus, welcher in einer Rotationsrichtung um die Längsachse (3) kein Drehmoment überträgt, und/oder ein Planetengetriebe zur Untersetzung des Drehmoments umfasst.

9. Vorrichtung nach einem der Ansprüche 1 bis 8, **dadurch gekennzeichnet, dass** mindestens am Einsatz (17) und am Handgriff (9) RFID-Tags (26) angebracht sind.

10. Vorrichtung nach einem der Ansprüche 1 bis 9, **dadurch gekennzeichnet, dass** die Ladeeinrichtung (16) eine Druckfeder (27) und ein Anpressteil (28) umfasst, wobei das Anpressteil (28) mittels Federkraft der Druckfeder (27) gegen ein vorderes Ende der Drehmomentübertragungseinheit (13) gedrückt wird.

11. Vorrichtung nach Anspruch 10, **dadurch gekennzeichnet, dass** der Schaft (2) einen entlang der Längsachse (3) verschiebbaren Markierungsring (29) umfasst, welcher mit dem Anpressteil (28) verbunden ist, wobei der Handgriff (9) entlang der Längsachse (3) voneinander beabstandete seitliche Öffnungen (30) umfasst, so dass der Markierungsring (29) je nach Position durch eine der seitlichen Öffnungen (30) sichtbar ist.

12. Vorrichtung nach Anspruch 11, **dadurch gekennzeichnet, dass** der Markierungsring (29) ein lichtleitendes und/oder lumineszierendes Material umfasst.

13. Kombination umfassend eine Vorrichtung nach Anspruch 11 oder 12 und eine elektronische Lesevorrichtung (31), mittels welcher die bei der Verwendung der Vorrichtung (1) an der Drehmomentübertragungseinheit (13) verbleibende Anzahl Drehmomentbegrenzungselemente (14) detektierbar ist.

14. Kit umfassend eine Vorrichtung gemäss einem der Ansprüche 1 bis 12 und eine Mehrzahl unterschiedlicher Sets, wobei jedes Set folgendes umfasst:
ein untrennbar mit einem Schaft (2) verbundenes Werkzeug (7), welches an einem freie Ende (33) Mittel zur Übertragung eines Drehmoments auf ein chirurgisches Implantat oder Werkzeug aufweist; und
eine Drehmomentübertragungseinheit (13), deren Sollbruchstellen (15) das zu übertragende Drehmoment auf einen Wert begrenzen, welcher den Mitteln zur Übertragung eines Drehmoments am freien Ende (33) des Werkzeugs (7) angepasst ist und/oder für eine beabsichtigte Verwendung der Vorrichtung (1) wählbar ist;
wobei
in der Kavität (8) des Schaftes (2) und an der Drehmomentübertragungseinheit (13) für jedes Set unterschiedliche, komplementär ausgebildete Positioniermittel (24a, 24b) angeordnet sind, welche nur in dieser Kombination von Drehmomentübertragungseinheit (13) und Schaft (2) miteinander in Eingriff bringbar sind.

15. Verfahren zur Montage der Vorrichtung gemäss einem der Ansprüche 1 bis 12 **gekennzeichnet durch** die Schritte:
a) Einsetzen des Schaftes (2) zusammen mit dem am vorderen Ende (5) des Schaftes (2) angeordneten Werkzeug (7) in den Handgriff (9), wodurch eine erste Baugruppe gebildet wird;
b) Verbinden des Einsatzes (17) mit einer ungebrauchten Drehmomentübertragungseinheit (13), wobei die ersten Befestigungsmittel (18) am hintersten Drehmomentbegrenzungselement (14) der ungebrauchten Drehmomentübertragungseinheit (13) mit den zweiten Befestigungsmitteln (19) am Einsatz (17) in Eingriff gebracht werden und die Mittel (20) zur drehfesten Verbindung des Einsatzes (17) mit dem jeweils hintersten Drehmomentbegrenzungselement (14) in Eingriff gebracht werden, wodurch eine zweite Baugruppe gebildet wird;
c) Einsetzen der zweiten Baugruppe in die erste Baugruppe; und
d) Verriegeln des Einsatzes (17) im Handgriff (9).

## Claims

1. Device (1) for the transmission of a given torque to a surgical tool, the device (1) comprising:
A) a longitudinal shaft (2) with a longitudinal axis (3), a rear end (4) and a front end (5), wherein a surgical tool (7) is fastened or can be fastened at the front end (5) and the shaft (2) comprises a cavity (8) that is coaxial with the longitudinal axis (3) and is open at a rear end (4) of the shaft (2);
B) a handle (9) with a first end (10), a second end (11) and a hollow space (12) penetrating the handle (9) along the longitudinal axis (3) from the first end (10) to the second end (11), the shaft (2) being supported in the hollow space (12) of the handle (9), at least on a portion of its length, such that it is rotatable around the longitudinal axis (3);
C) a torque transmission unit (13) with a plurality of torque-limiting elements (14), which are arranged behind one another along the longitudinal axis (3) and are connected to one another in each case via a predetermined rupture point (15), wherein the torque transmission unit (13) is arranged in the cavity (8) of the shaft (2) so as to be rotationally fixed but displaceable coaxially with the longitudinal axis (3), such that a rearmost torque-limiting element (14) protrudes beyond the rear end (4) of the shaft (2) and is connected to the handle (9) in a rotationally fixed manner, wherein if a given torque is exceeded, the rearmost torque-limiting element (14) is separated off at the predetermined rupture point (15); and
wherein
D) the shaft (2) comprises a loading device (16) via which the torque transmission unit (13) is axially displaceable relative to the longitudinal axis (3) toward the rear end (4) of the shaft (2), such that following separation of the rearmost torque-limiting element (14), the axially adjacent torque-limiting element (14) protrudes beyond the rear end (4) of the shaft (2) and is connected, in its turn, in a rotationally fixed manner, to the handle (9) as the rearmost torque-limiting element (14),
**characterised in that**
E) the device (1) comprises an insert (17) which, in a mounted state of the device (1), is introduced into the hollow space (12) of the handle (9) and is releasably fixed to the handle (9) axially and rotationally, wherein
F) the rearmost torque-limiting element (14) of the unused torque transmission unit (13) comprises first fastening members (18) and the insert (17) comprises second fastening members (19), which may be brought to engage with one another for fastening the unused torque transmission unit (13) at the insert (17); and
G) the insert (17) comprises means (20) for a rotationally fix connection with the respectively rearmost torque-limiting element (14).

2. Device according to claim 1,
**characterised in that** the insert (17) comprises a chamber (21) for receiving torque-limiting elements (14) separated off during usage of the device (1).

3. Device according to claim 1 or 2,
**characterised in that** the second fastening members (19) arranged at the insert (17) can be brought into a first position and into a second position, wherein in the first position an unused torque transmission unit (13) can be fixed at the insert (17) releasably, and in the second position the insert (17) can be fixed at the handle (9) axially and rotationally.

4. Device according to one of claims 1 to 3,
**characterised in that** the second fastening members (19) comprise a push button (22) and a spring (23), and that the push button (22) can be brought into the first position against the spring force of the spring (23), and can be brought into the second position by means of the spring force of the spring (23).

5. Device according to one of claims 1 to 4,
**characterised in that** the tool (7) is inseparably connected with the shaft (2), and in the cavity (8) and at the torque transmission unit (13) there are complementarily realized positioning means (24a, 24b) arranged, which can be brought to engage with each other only in a defined combination of torque transmission unit (13) and shaft (2).

6. Device according to one of claims 1 to 5,
**characterised in that** the torque-limiting elements (14) have a non-circular cross-section area orthogonally to the longitudinal axis (3),
wherein the means (20) for the rotationally fix connection of the insert (17) with the respectively rearmost torque-limiting element (14) have a pass-through opening (25) that has a non-circular cross-section area orthogonally to the longitudinal axis (3) which is an exact fit with the cross-section area of the torque-limitng elements (14), such that the pass-through opening (25) can be brought to engage with the respectively rearmost torque-limiting element (14) in a form-fit manner.

7. Device according to claim 6,
**characterised in that** at least in a region of the rear end (4) of the shaft (2), the cavity (8) in the shaft (2) has a non-circular cross-section area, such that the cavity (8) can be brought to engage with one or several torque-limiting elements (14) in a form-fit manner.

8. Device according to one of claims 1 to 7,
**characterised in that** the shaft (2) has a ratchet mechanism which does not transfer a torque in a rotation direction around the longitudinal axis (3), and/or has a planetary gear for a reduction of the torque.

9. Device according to one of claims 1 to 8,
**characterised in that** RFID tags (26) are attached at least at the insert (17) and at the handle (9).

10. Device according to one of claims 1 to 9,
**characterised in that** the loading device (16) comprises a compression spring (27) and a pressing-on member (28), wherein the pressing-on member (28) is pressed against a front end of the torque transmission unit (13) by a spring force of the compression spring (27).

11. Device according to claim 10,
**characterised in that** the shaft (2) comprises a marking ring (29), which is displaceable along the longitudinal axis (3) and is connected to the pressing-on member (28), the handle (9) having sidewise openings (30) spaced apart from one another along the longitudinal axis (3), such that the marking ring (29) is visible through one of the sidewise openings (30) depending on a position.

12. Device according to claim 11,
**characterised in that** the marking ring (29) comprises a fibre-optic material and/or a luminescent material.

13. Combination, comprising a device according to claim 11 or 12 and an electronic reading device (31) that allows detecting the number of torque-limiting elements (14) remaining at the torque transmission unit (13) when the device (1) is used.

14. Kit comprising a device according to one of claims 1 to 12, and a plurality of different sets, each set comprising the following:
a tool (7) that is inseparably connected with a shaft (2) and has at a free end (33) means for transmitting a torque to a surgical implant or tool; and
a torque transmission unit (13), whose predetermined rupture points (15) limit the torque to be transmitted to a value which is adapted to the means for transmitting a torque at the free end (33) of the tool (7) and/or is selectable for an intended utilisation of the device (1),
wherein
for each set different, complementarily realized positioning means (24a, 24b) are arranged in the cavity (8) of the shaft (2) and at the torque transmission unit (13), wherein said positioning means (24a, 24b) can be brought to engage with one another only in this combination of torque transmission unit (13) and shaft (2).

15. Method for an assembly of the device according to one of claims 1 to 12, **characterized by** the steps:
a) inserting the shaft (2) together with the tool (7) that is arranged at the front end (5) of the shaft (2) into the handle (9), resulting in the formation of a first assembly group;
b) connecting the insert (17) with an unused torque transmission unit (13), wherein the first fastening members (18) at the rearmost torque-limiting element (14) of the unused torque transmission unit (13) are brought to engage with the second fastening members (19) at the insert (17), and
the means (20) for a rotationally fix connection of the insert (17) with the respectively rearmost torque-limiting element (14) are brought into engagement, resulting in the formation of a second assembly group;
c) inserting the second assembly group into the first assembly group; and
d) locking the insert (17) in the handle (9).

## Revendications

1. Dispositif (1) pour transmettre un couple spécifié sur un outil chirurgical, le dispositif (1) comprenant :
A) un manche longitudinal (2) ayant un axe longitudinal (3), une extrémité arrière (4) et une extrémité avant (5), où un outil chirurgical (7) est fixé ou peut être fixé sur l'extrémité avant (5) et le manche (2) comprend une cavité (8) qui est coaxiale avec l'axe longitudinal (3) et est ouverte à l'extrémité arrière (4) du manche (2) ;
B) une poignée (9) ayant une première extrémité (10), une deuxième extrémité (11) et un espace creux (12) pénétrant la poignée (9) le long de l'axe longitudinal (3) de la premiére extrémité (10) jusqu'à la deuxième extrémité (11), le manche (2) étant supporté, au moins en une partie de sa longueur, dans l'espace creux (12) de la poignée (9) d'une telle manière qu'il peut être tourné autour de l'axe longitudinal (3) ;
C) une unité de transmission-couple (13), avec une pluralité des éléments de limitation-couple (14) disposés l'un derrière l'autre le long de l'axe longitudinal (3) et reliés l'un à l'autre respectivement via un point de rupture (15),
où l'unité de transmission-couple (13) est disposée dans la cavité (8) du manche (2) d'une telle manière qu'elle soit déplaçable fixement en rotation mais coaxialement, en sorte qu'un élément de limitation-couple le plus arrière (14) saille au-delà de l'extrémité arrière (4) du manche (2) et soit relié fixement en rotation avec la poignée (9), où l'élément de limitation-couple le plus arrière (14) est séparé dans le point de rupture (15) si un couple spécifié est dépassé ; et où
D) le manche (2) comprend un dispositif de chargement (16) par le biais duquel l'unité de transmission-couple (13) peut être déplacée par rapport à l'axe longitudinal (3) axialement vers l'extrémité arrière (4) du manche (2), en sorte que suivant la séparation de l'élément de limitation-couple le plus arrière (14), l'élément de limitation-couple avoisinant axialement (14) saille au-delà de l'extrémité arrière (4) du manche (2) et est à son tour relié, fixement en rotation, à la poignée (9) comme l'élément de limitation-couple le plus arrière (14),
**caractérisé en ce que**
E) le dispositif (1) comprend un insert (17) qui est en état monté du dispositif (1) introduit dans l'espace creux (12) de la poignée (9) et est fixé à la poignée (9) axialement et rotativement de façon relâchable, où
F) l'élément de limitation-couple le plus arrière (14) de l'unité de transmission-couple non-utilisée (13) comprend des premiers moyens de fixement (18) et l'insert (17) comprend des deuxièmes moyens de fixement (19), lesdits moyens de fixement (17, 18) pouvant être mis en engrènement les uns avec les autres pour le fixement de l'unité de transmission-couple non-utilisée (13) à l'insert (17) ; et
G) l'insert (17) comprend des moyens (20) pour une liaison fixe en rotation avec l'élément de limitation-couple (14) qui est respectivement le plus arrière.

2. Dispositif selon la revendication 1,
**caractérisé en ce que** l'insert (17) comprend une chambre (21) pour recevoir des éléments de limitation-couple (14) séparées lors d'une utilisation du dispositif (1).

3. Dispositif selon la revendication 1 ou 2,
**caractérisé en ce que** les deuxièmes moyens de fixement (19) disposés sur l'insert (17) peuvent être mis dans une première position et dans une deuxième position, où dans la première position une unité de transmission-couple non-utilisée (13) peut être fixée à l'insert (17) relâchablement et dans la deuxième position l'insert (17) peut être fixé sur la poignée (9) axialement et rotativement.

4. Dispositif selon l'une des revendications 1 à 3,
**caractérisé en ce que** les deuxièmes moyens de fixement (19) comprennent un bouton poussoir (22) et un ressort (23), et que le bouton poussoir (22) peut être mis dans la première position contre la force-ressort du ressort (23) et peut être mis dans la deuxième position par le biais de la force-ressort du ressort (23).

5. Dispositif selon l'une des revendications 1 à 4,
**caractérisé en ce que** l'outil (7) est relié au manche (2) inséparement et que dans la cavité (8) et sur l'unité de transmission-couple (13) des moyens de positionnement (24a, 24b) sont disposés qui sont réalisés complémentairement et peuvent être mis en engrènement les uns avec les autres seulement dans une combination spécifique d'unité transmission-couple (13) et manche (2).

6. Dispositif selon l'une des revendications 1 à 5,
**caractérisé en ce que** les éléments de limitation-couple (14) ont orthogonalement à l'axe longitudinal (3) une surface de coupe transversale qui n'est pas circulaire, où les moyens (20) pour la liaison fixe en rotation de l'insert (17) avec l'élément de limitation-couple le respectivement plus arriére (14) comprennent une ouverture de passage (25) ayant orthogonalement à l'axe longitudinal (3) une surface de coupe transversale qui n'est pas circulaire et qui est précisément sur mesure avec la surface de coupe transversale des éléments de limitation-couple (14),
en sorte que l'ouverture de passage (25) puisse être mis en engrènement par liaison en forme avec l'élément de limitation-couple le respectivement plus arrière (14).

7. Dispositif selon la revendication 6,
**caractérisé en ce que** la cavité (8) dans le manche (2) comporte, au moins dans une zone de l'extrémité arrière (4) du manche (2), une surface de coupe transversale qui n'est pas circulaire, en sorte que la cavité (8) puisse être mis, par liaison en forme, en engrènement avec un ou plusieurs élément(s) de limitation-couple (14).

8. Dispositif selon l'une des revendications 1 à 7,
**caractérisé en ce que** le manche (2) comporte un mécanisme à cliquet qui ne transfert aucun torque dans une direction rotative autour de l'axe longitudinal (3), et/ou comporte un engrenage planétaire pour une réduction en vitesse du torque.

9. Dispositif selon l'une des revendications 1 à 8,
**caractérisé en ce que** des tags RFID (26) sont attachés au moins à l'insert (17) et à la poignée (9).

10. Dispositif selon l'une des revendications 1 à 9,
**caractérisé en ce que** le dispositif de chargement (16) comprend un ressort à pression (27) et un élément pression-contact (28), l'élément pression-contat (28) étant pressé contre une extrémité avant de l'unité de transmission-couple (13) par la force-ressort du ressort à pression (27).

11. Dispositif selon la revendication 10,
**caractérisé en ce que** le manche (2) comprend un anneau-marqueur (29) qui est déplaçable le long de l'axe longitudinal (3) et est relié avec l'élément pression-contact (28), la poignée (9) comprenant des apertures latérales (30) qui sont à l'écart le long de l'axe longitudinal (3), en sorte que l'anneau-marqueur (29) soit visible par l'une des apertures latérales (30) en fonction d'une position.

12. Dispositif selon la revendication 11,
**caractérisé en ce que** l'anneau-marqueur (29) comprend un matériau fibre-optique et/ou luminescent.

13. Combination, comprenant un dispositif selon la revendication 11 ou 12, et un appareil électronique de lecture (31), par le biais duquel le nombre des éléments de limitation-couple (14) restant sur l'unité de transmission-couple (13) lors de l'utilisation du dispositif (1).

14. Kit, comprenant un dispositif selon l'une des revendications 1 à 12 et une pluralité de sets différents, chacun des sets comprenant le suivant :
un outil (7) qui est relié à un manche (2) inséparablement et comprend à une extrémité libre (33) des moyens de transmission-couple sur un implant chirurgical ou outil chirurgical ; et
une unité de transmission-couple (13), les points de rupture (15) de laquelle limitent le couple qui est à transmettre à une valeur adaptée aux moyens de transmission-couple sur l'extrémité libre (33) de l'outil (7) et/ou peut être sélectionnée librement pour une utilisation prévue du dispositif (1) ;
où pour chaque set, dans la cavité (8) du manche (2) et sur l'unité de transmission-couple (13), des moyens à positionnement différents (24a, 24b), réalisés complémentairement, sont disposés, qui peuvent être mis en engrènement l'un avec l'autre seulement dans cette combination d'unité de transmission-couple (13) et manche (2).

15. Procédé pour le montage du dispositif selon l'une des revendications 1 à 12, **caractérisé par** les étapes :
a) insérer le manche (2) conjointement avec l'outil (7) disposé à l'extrémité avant (5) du manche (2) dans la poignée (9), moyennant quoi un premier module de construction est formé ;
b) relier l'insert (17) avec une unité de transmission-couple non-utilisée (13), où les premiers moyens de fixement (18) sur l'élément de limitation-couple le plus arrière (14) de l'unité de transmission-couple non-utilisée (13) sont mis en engrènement avec les deuxièmes moyens de fixement (19) sur l'insert (17), et
les moyens (20) pour la liaison fixe en rotation de l'insert (17) sont mis en engrènement avec l'élément de limitation-couple le respectivement plus arrière (14), moyennant quoi un deuxième module de construction est formé ;
c) insérer le deuxième module de construction dans le premier module de construction ; et
d) verrouiller l'insert (17) dans la poignée (9).
